# EUROPEAN PATENT SPECIFICATION

(11) **EP 3 141 273 B1**
(45) Date of publication and mention of the grant of the patent: **30.10.2019**
(21) Application number: 16195495.3
(22) Date of filing: 04.04.2012
(51) Int. Cl.: A61M 1/36

(54) **FLUID-BASED HYPER/HYPOTHERMIA SYSTEM WITH TEMPERATURE CONTROL DEVICE**
HYPER-/HYPOTHERMIESYSTEM AUF FLUIDBASIS MIT TEMPERATURREGELUNGSVORRICHTUNG
SYSTÈME D'HYPER/HYPOTHERMIE À BASE DE FLUIDE AVEC DISPOSITIF DE RÉGULATION DE LA TEMPÉRATURE

(30) Priority: 08.04.2011 DE 102011016508
(43) Date of publication of application: 15.03.2017
(62) Divisional of application: 12716297.2
(73) Proprietor: Sorin Group Deutschland GmbH, 80939 München (DE)
(72) Inventor: Knott, Erwin, 85586 Poing (DE); Fronhöfer, Manfred, 81379 München (DE)
(74) Representative: Hoffmann Eitle

(56) References cited:
- EP-A1- 1 970 080
- WO-A1-97/06840
- WO-A1-2006/063080
- US-A- 6 156 007
- US-A1- 2007 020 142
- US-A1- 2010 143 192
- US-B2- 6 581 403

## Description

The invention relates to a fluid-based hyper/hypothermia system.

A fluid-based hyper/hypothermia system is disclosed, for example, in DE 696 34 572 T2.

EP 1 970 080 A1 relates to a device for adjusting the temperature of a physiological fluid. The device includes: a casing, a first heat energy generating unit, a heat energy receiving unit through which the fluid flows along a passage from a fluid inlet to a fluid outlet and which consists of a flat body having a first large surface which is made from a heat conductor material, and a control unit for controlling at least the first generating unit. Here, the first generating unit includes first units of Peltier cells and at least one first contact plate which is made from a heat conductor material and which is placed in contact with a first side of the units of Peltier cells. In addition, the receiving unit is removably installed in the first generating unit such that it is in contact with the first contact plate.

US 6 156 007 A teaches an apparatus for use in whole body hypothermia including a dialyzer having a blood side and a dialysate side separated by one or more dialysis membranes. A blood pump associated with the blood circuit established on the blood side circulates blood through the dialyzer at high rates advantageous for hyperthermia treatments. A bypass is included whereby circulated blood can bypass the dialyzer. The bypass aids in preventing blood-side tensioning of dialyzer membranes thereby allowing proper membrane movement to assist in mixing a sorbent suspension circulated on the dialysate side of the dialyzer. A sorbent suspension composition for use as a dialysate in hemodialysis, especially when performed in conjunction with whole body hyperthermia, includes water, a surface adsorptive agent such as charcoal, a cation exchanger, and precipitated calcium phosphate. The precipitated calcium phosphate serves as a reservoir for calcium and phosphate ions to assist in controlling patient blood chemistries.

WO 2006/063080 A1 discloses systems and methods for temperature adjustment using bodily fluids as a thermic medium. In particular, an apparatus is described for affecting the temperature of a bodily organ using a bodily fluid as a thermic medium. The apparatus can include a fluid-drawing device such as a needle, an extra-bodily fluid pathway that can comprise a flexible medical tube, a temperature-affecting device such as a Peltier cooler or resistive heater, a pump such as a peristaltic pump, and a fluid-insertion device. The temperature affecting device can be in thermal contact with the extra-bodily fluid pathway.

US 6 581 403 B2 shows a heating/cooling system for indwelling heat exchange catheter, wherein the cooling system includes a heat exchange bath that is configured to receive a conduit that carries saline to and from the catheter. A heating/cooling fluid is in the bath and exchanges heat with the saline. The heating/cooling fluid flows through a heat exchanger that includes a refrigerant and a variable speed DC compressor for removing heat from the refrigerant. A coolant pump circulates the heating/cooling fluid between the heat exchanger and the heat exchange bath.

Fluid-based hyper/hypothermia systems which use a temperature-controlled fluid to raise the temperature of a human or animal body, body part or organ to above the normal core body temperature or to lower it to below the normal core body temperature require a temperature control device that provides a temperature-controlled fluid, by means of which the desired change in body temperature can be effected. The temperature of the fluid must be controlled in the temperature control device in accordance with the quantity of heat to be supplied to or removed from the body, i.e. it must be heated or cooled and then maintained at a predetermined temperature.

In order to heat or cool the fluid in the temperature control device, energy is required that is provided as a general rule by the local power network. Thus, a conventional temperature control device comprises a power supply which allows the temperature control device to be connected to the local power network. Both the power supply as well as numerous individual components of the temperature control device must be adapted to the local power network. Since there are different local power networks in different regions of the world, the region of the world in which the temperature control device is ultimately supposed to be used and the specifications of the local power network according to which the power supply of the temperature control device and the temperature control device itself have to be configured must, with a considerable amount of effort, always be taken into consideration when constructing a temperature control device for hyper/hypothermia applications.

The aim of the invention is to simplify this aspect of the construction of a temperature control device and to provide a hyper/hypothermia system that can be used in different regions of the world owing to a simple adaptation.

This aim is achieved by a fluid-based hyper/hypothermia system having the features specified in patent claim 1. Advantageous embodiments are revealed by the sub-claims.

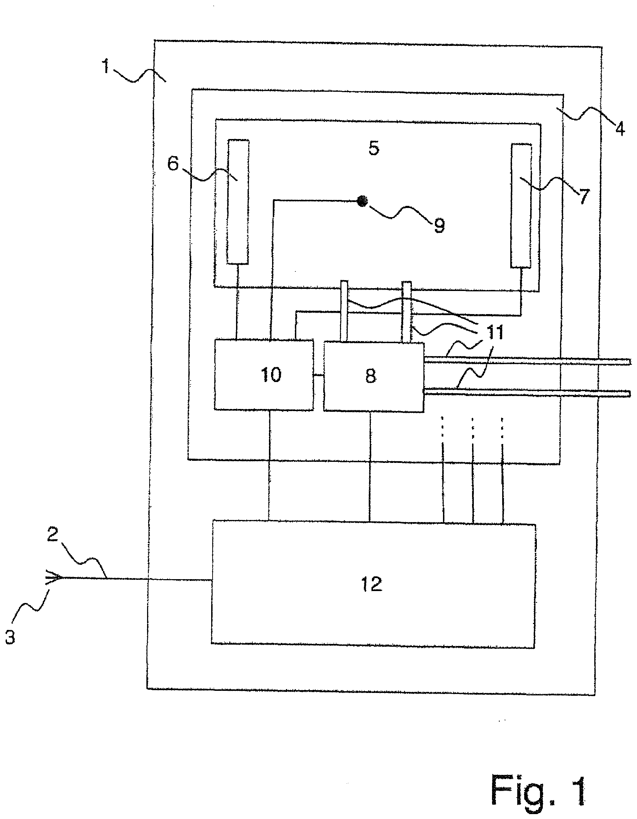

The invention is explained in the following by means of an embodiment and with reference to Fig. 1.
- Fig. 1: shows an embodiment of a temperature control device of a fluid-based hyper/hypothermia system according to the invention;
- Fig. 2: shows a further embodiment of the temperature control device of a fluid-based hyper/hypothermia system according to the invention.

The embodiment of a temperature control device 1 of a fluid-based hyper/hypothermia system according to the invention is shown in Fig. 1 and comprises a connection unit 2 for connecting the device to a local power network 3. In Germany, this is the general AC network of 220/380 V at 50 Hz, in Japan it is an AC network of 100 V at, for example, 60 Hz, and in the USA it is, in turn, an AC network of 120 V at 60 Hz. These differences, in particular also in the frequencies of the local power networks, lead to differences in the leakage currents which result from the change of the connected alternating current over time and with regard to which strict guidelines apply, in particular for medical-technical systems in a surgical environment since the effect of electrical currents in the case, for example, of open heart surgeries must remain minimal. In order to minimise these leakage currents, the electric lines in conventional temperature control devices must have certain insulations. This leads to increased material costs since in particular the insulation can age and must then be replaced if the guidelines with respect to the leakage currents are no longer met.

The temperature control device of a fluid-based hyper/hypothermia system according to the invention is connected to the power network 3 via the connection unit 2 and can draw the power required to control the temperature of the fluid from the power network.

The temperature control of the fluid is carried out by means of a fluid temperature control unit 4 which comprises the components that are required for heating or cooling the fluid. These normally include a fluid container 5, a heater 6, a cooler 7, a supply pump 8, a temperature sensor 9 and a temperature controller 10, which are shown in Fig. 1 merely in schematic form and as an example of the components of the fluid temperature control unit 4. In the present embodiment, the supply pump 8 works, for example, with a direct current motor whereas the cooler 7 has a direct current compressor. Also shown by way of an example are pipelines 11, via which the pump 8 removes the fluid from the fluid container 5 and conveys it to the outside such that it can be used in the hyper/hypothermia system, or via which the fluid is conveyed out of the hyper/hypothermia system back into the fluid container 5. The pump can also be provided in the hyper/ hypothermia system such that it can be omitted from the fluid temperature control unit 4 of a temperature control device 1 of a fluid-based hyper/hypothermia system as according to the invention. Depending on the hyper/ hypothermia system in which the temperature control device 1 is used, other components, such as a stirrer for the fluid in the fluid container 5, may be added to/omitted from the fluid temperature control unit 4.

In order to supply power to the electrical consumers, i.e., for example, the heater 6, the cooler 7, the supply pump 8 and the temperature controller 10, of the fluid temperature control unit of a temperature control device 1 of a fluid-based hyper/hypothermia system according to the invention, a power supply unit 12 is provided according to the invention, via which all of the electrical consumers of the fluid temperature control unit 4 are electrically supplied with constant connected loads, i.e. irrespective of the local power network. According to the invention, direct current is supplied, for example with a supply voltage of 48 V and a power of up to 3.5 kW. This means that according to the invention, the consumers of the fluid temperature control unit 4 which are supplied via the power supply unit are all not directly connected to the power network 3 and do not have to be designed for this power network, but are rather all supplied with direct current by the power supply unit 12 according to the invention. Different consumers can thereby be supplied with different voltages/powers which are provided by the power supply unit 12 according to the invention, and this is indicated in Fig. 1 by the connections between the power supply unit 12 and the fluid temperature control unit 4 which are dashed at one end. The power supply unit 12 according to the invention thereby performs adaptation to the local power network and conversion to a power supply with constant connected loads.

By means of the power supply unit of a fluid-based hyper/hypothermia system according to the invention, it is achieved that the temperature control device can be adapted to the conditions of a regional power network with an acceptable amount of effort. The adaptation to the local power network of the region in which the device is to be used is achieved by an appropriate design of the power supply unit, which, on the side facing the connection unit, must be designed for connection to the local power network, whereas on the side facing the fluid temperature control unit, the aforementioned uniform power supply with direct current is ensured irrespective of the local power network.

Suitable as the power supply unit of a fluid-based hyper/hypothermia system according to the invention are standard power supplies (also switched-mode power supplies) that provide, as standard, one or more supply voltages which are required by the fluid temperature control unit so that the temperature of the fluid can be controlled.

It is achieved with the power supply unit of a fluid-based hyper/hypothermia system as provided according to the invention that the fluid temperature control unit is electrically separate from the local power network. As a result, an improved electrical decoupling of the fluid temperature control unit from the power network is achieved, which has a positive effect on use in hyper/hypothermia systems since network feedback and leakage currents can be reduced. Attention must be paid in this respect to the fact that as medical-technical systems, hyper/hypothermia systems are subject to particularly critical specifications, which is why the decoupling of the fluid temperature control unit from the local power network that is achieved by the power supply unit of a fluid-based hyper/hypothermia system according to the invention is to be regarded as positive.

Supplying the electrical consumers of the fluid temperature control unit with direct current offers the possibility of a more precise control during operation since a precise power control for each individual electrical consumer can take place, for example, with the aid of inverters. This is true not only for the heater/cooler of the fluid temperature control unit but also for the pumps which are generally electromotively driven. Overall, the clearly improved controllability of the temperature control device of a fluid-based hyper/hypothermia system according to the invention leads to a noise-reducing scenario when used in a hyper/hypothermia treatment.

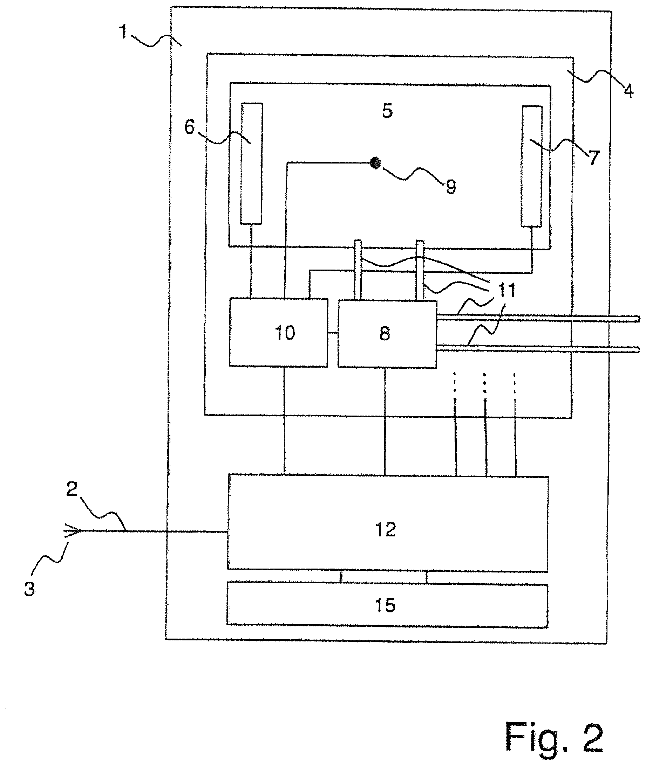

The further embodiment of a temperature control device 1 of a fluid-based hyper/hypothermia system according to the invention which is shown in Fig. 2 additionally comprises a battery 15 for supplying the electrical consumers of the fluid temperature control unit 4 with power. The battery 15 is connected to the power supply unit 12 and is charged by this unit when the supply of power occurs via the power network. As a result, a fail-safe supply of direct current to the electrical consumers can be effected in the case than, for example, the local power network is subject to fluctuations or fails completely, since in this case the battery 15 can supply the power supply unit 12. Thus, designing the temperature control device with direct current consumers makes it possible to ensure the continuous operation of the temperature control device in a surgical environment.

## Claims

1. A fluid-based hyper/hypothermia system which uses a temperature-controlled fluid to raise a temperature of a human or animal body to above a normal core body temperature or to lower the temperature below the normal core body temperature, said fluid-based hyper/hypothermia system including a temperature control device (1) comprising:
a connection unit (2), configured to connect the device to a local power network (3); and
a fluid temperature control unit (4), configured to heat the fluid in an open heart surgery procedure to above the normal core body temperature or to cool the fluid in an open heart surgery procedure to below the normal core body temperature,
including a power supply unit (12) that is configured to supply all electrical consuming components of the fluid temperature control unit (4) with direct current,
**characterized in that**:
the power supply unit (12) is further configured to perform conversion to a power supply with constant connected loads irrespective of the local power network, and is configured to supply different electrical consuming components of the electrical consuming components with different voltages and powers, wherein the electrical consuming components are all not directly connected to a power network (3).

2. The fluid-based hyper/hypothermia system according to claim 1, wherein the power supply unit (12) is a switched-mode power supply.

3. The fluid-based hyper/hypothermia system according to one of claims 1 to 2, wherein the electrical consuming components of the fluid temperature control unit (4) comprise a direct current motor.

4. The fluid-based hyper/hypothermia system according to one of claims 1 - 3, wherein the electrical consuming components of the fluid temperature control unit comprise a direct current compressor.

5. The fluid-based hyper/hypothermia system according to one of claims 1 to 4, wherein the temperature control device (1) comprises a battery (15) for supplying the electrical consuming components of the fluid temperature control unit (4), said battery being connected to the power supply unit (12) such as to be charged by the power supply unit or to supply the power supply unit in case of failure of the local power network (3).

## Patentansprüche

1. Fluidbasiertes Hyper-/Hypothermie-System, das ein temperaturgesteuertes Fluid verwendet, um eine Temperatur eines menschlichen oder tierischen Körpers auf über eine normale Körperkerntemperatur zu erhöhen oder um die Temperatur unter die normale Körperkerntemperatur zu senken, wobei das fluidbasierte Hyper-/Hypothermie-System eine Temperatursteuervorrichtung (1) einschließt, umfassend:
eine Anschlusseinheit (2), die konfiguriert ist, um die Vorrichtung an ein örtliches Stromnetz (3) anzuschließen; und
eine Fluidtemperatur-Steuereinheit (4), die konfiguriert ist, um das Fluid in einer Operationsprozedur am offenen Herzen auf über die normale Körperkerntemperatur zu erwärmen oder um das Fluid in einer Operationsprozedur am offenen Herzen auf unter die normale Körperkerntemperatur abzukühlen,
einschließlich einer Stromversorgungseinheit (12), die konfiguriert ist, um alle elektrischen verbrauchenden Komponenten der Fluidtemperatur-Steuereinheit (4) mit Gleichstrom zu versorgen,
**dadurch gekennzeichnet, dass**:
die Stromversorgungseinheit (12) weiter konfiguriert ist, um Umrichten an einer Stromversorgung mit festen angeschlossenen Lasten unabhängig von dem örtlichen Stromnetz durchzuführen, und konfiguriert ist, um unterschiedliche elektrische verbrauchende Komponenten der elektrischen verbrauchenden Komponenten mit unterschiedlichen Spannungen und Strömen zu versorgen, wobei die elektrischen verbrauchenden Komponenten alle nicht direkt an ein Stromnetz (3) angeschlossen sind.

2. Fluidbasiertes Hyper-/Hypothermie-System nach Anspruch 1, wobei die Stromversorgungseinheit (12) ein Schaltnetzteil ist.

3. Fluidbasiertes Hyper-/Hypothermie-System nach einem der Ansprüche 1 bis 2, wobei die elektrischen verbrauchenden Komponenten der Fluidtemperatur-Steuereinheit (4) einen Gleichstrommotor umfassen.

4. Fluidbasiertes Hyper-/Hypothermie-System nach einem der Ansprüche 1-3, wobei die elektrischen verbrauchenden Komponenten der Fluidtemperatur-Steuereinheit einen Gleichstromkompressor umfassen.

5. Fluidbasiertes Hyper-/Hypothermie-System nach einem der Ansprüche 1 bis 4, wobei die Temperatursteuervorrichtung (1) eine Batterie (15) zur Versorgung der elektrischen verbrauchenden Komponenten der Fluidtemperatur-Steuereinheit (4) umfasst, wobei die Batterie an die Stromversorgungseinheit (12) angeschlossen ist, um durch die Stromversorgungseinheit aufgeladen zu werden oder um die Stromversorgungseinheit im Falle eines Ausfalls des örtlichen Stromnetzes (3) zu versorgen.

## Revendications

1. Système d'hyper/hypothermie à base de fluide qui utilise un fluide régulé en température pour augmenter une température d'un corps humain ou animal au-dessus d'une température corporelle normale ou pour abaisser la température en dessous de la température corporelle centrale normale, ledit système d'hyper/hypothermie à base de fluide incluant un dispositif de régulation de température (1) comprenant :
une unité de connexion (2), configurée pour connecter le dispositif à un réseau électrique local (3) ; et
une unité de régulation de température de fluide (4), configurée pour chauffer le fluide lors d'une opération chirurgicale à cœur ouvert au-dessus de la température corporelle centrale normale ou pour refroidir le fluide lors d'une opération chirurgicale à cœur ouvert en dessous de la température corporelle centrale normale,
incluant une unité d'alimentation électrique (12) qui est configurée pour alimenter l'ensemble des consommateurs électriques de l'unité de régulation de température de fluide (4) en courant continu,
**caractérisé en ce que** :
l'unité d'alimentation électrique (12) est en outre configurée pour effectuer une conversion en une alimentation électrique ayant des charges connectées constantes indépendamment du réseau électrique local, et est configurée pour alimenter différents consommateurs électriques des consommateurs électriques avec différentes tensions et puissances, dans lequel les consommateurs électriques sont tous non directement connectés à un réseau d'alimentation (3).

2. Système d'hyper/hypothermie à base de fluide selon la revendication 1, dans lequel l'unité d'alimentation électrique (12) est une alimentation électrique en mode commuté.

3. Système d'hyper/hypothermie à base de fluide selon l'une des revendications 1 à 2, dans lequel les consommateurs électriques de l'unité de régulation de température de fluide (4) comprennent un moteur à courant continu.

4. Système d'hyper/hypothermie à base de fluide selon l'une des revendications 1 à 3, dans lequel les consommateurs électriques de l'unité de régulation de température de fluide comprennent un compresseur à courant continu.

5. Système d'hyper/hypothermie à base de fluide selon l'une des revendications 1 à 4, dans lequel le dispositif de régulation de température (1) comprend une batterie (15) pour alimenter les consommateurs électriques de l'unité de régulation de température de fluide (4), ladite batterie étant connectée à l'unité d'alimentation électrique (12) de manière à être chargée par l'unité d'alimentation électrique ou à alimenter l'unité d'alimentation électrique en cas de panne du réseau électrique local (3).
